# EUROPEAN PATENT SPECIFICATION

(11) **EP 2 012 873 B1**
(45) Date of publication and mention of the grant of the patent: **03.10.2018**
(21) Application number: 07724506.6
(22) Date of filing: 24.04.2007
(51) Int. Cl.: A61N 1/36, A61F 2/14, A61F 9/08

(54) **ACTIVE SUB-RETINA IMPLANT**
AKTIVES SUBRETINALES IMPLANTAT
IMPLANT SOUS-RÉTINIEN ACTIF

(30) Priority: 28.04.2006 DE 102006021258
(43) Date of publication of application: 14.01.2009
(73) Proprietor: Retina Implant AG, 72770 Reutlingen (DE)
(72) Inventor: ZRENNER, Eberhart, 72076 Tübingen (DE); WROBEL, Walter-G, 72770 Reutlingen (DE); GEKELER, Florian, 72070 Tübingen (DE); HARSCHER, Alex, 72116 Mössingen (DE); WILKE, Robert, 72076 Tübingen (DE)
(74) Representative: Witte, Weller & Partner Patentanwälte mbB
(86) International application number: PCT/EP2007/003576
(87) International publication number: WO 2007/128404

(56) References cited:
- EP-A- 1 618 922
- WO-A-2005/000395
- US-A- 5 873 901
- US-A1- 2002 010 496
- US-A1- 2002 038 134
- US-A1- 2003 139 784
- SUANING G J; HALLUM L E; PRESTON P J; LOVELL N H: "An efficient multiplexing method for addressing large numbers of electrodes in a visual neuroprosthesis" CONF PROC IEEE ENG MED BIOL SOC., vol. 6, 2004, pages 4174-4177, XP002447117 United States

## Description

The present invention relates to an active retina implant for implantation into an eye, preferably into the subretinal space of an eye, with a multiplicity of stimulation electrodes that emit electrical stimulation signals to cells of the retina that are to be contacted, and with a multiplicity of image elements that convert incident light into the stimulation signals.

A retina implant of this kind is known from, for example, WO 2005/000395 A1, EP 1 618 922 A1 and the article by Suaning et al.: "An efficient multiplexing method for addressing large number of electrodes in a visual electrode", in CONF PROC IEEE ENG MED BIOL SOC., vol. 6, 2004, pages 4174-4177.

The known retina implant is used to counteract a loss of sight due to degeneration of the retina. The underlying concept is to implant a microelectronic stimulation chip into a patient's eye, thus replacing the lost sight by electrical excitation of nerve cells.

There are two different approaches to how retina prostheses of this kind can be configured. The subretinal approach uses a stimulation chip which is implanted into the subretinal space between the outer retina and the pigment epithelium of the retina and with which the ambient light impinging on an array of photodiodes integrated into the stimulation chip is converted into stimulation signals for nerve cells. This retina implant thus stimulates the remaining, intact neurons of the degenerated retina, that is to say horizontal cells, bipolar cells, amacrine cells, and possibly also ganglion cells. The visual image impinging on the array of photodiodes or more complex elements is thus converted into an electrical stimulation pattern that is then conveyed from the "natural computer" to the ganglion cells of the inner retina and from there is guided via the optic nerve into the visual cortex. In other words, the subretinal approach makes use of the natural circuitry of the previously present and now degenerated or absent photoreceptors with the ganglion cells in order to supply the visual cortex in the normal way with nerve impulses that correspond to the viewed image.

The energy for generating the electrical stimulation signals is either obtained from additionally irradiated invisible light or is delivered from an external source, for example via a coil or a cable.

By contrast, the epiretinal approach uses a device composed of an extraocular part and an intraocular part that communicate with one another in a suitable manner. The extraocular part comprises a camera and a microelectronic circuit for decoding incident light, that is to say the image information, and for transmitting it as a stimulation pattern to the intraocular part. The intraocular part contains an electrode array that contacts the neurons of the inner retina and thus directly stimulates the ganglion cells located there.

While the subretinal approach pursues the transmission of light and the stimulation of the retina *in situ*, the image information in the epiretinal approach has to be converted externally into a spatial and temporal stimulation pattern of electrical pulses to allow it to be "understood" by the visual cortex.

It is known, from many different publications, that the transmission of the stimulation signals from the stimulation electrodes to the contacted cells requires particular attention. This is because the coupling between a stimulation electrode and the contacted tissue is of a capacitive nature, such that only transient signals can be used for the stimulation. This capacitive coupling is based on the fact that, at the interface between electrode and electrolyte in the eye, a capacitance (Helmholtz double layer) is formed as a result of the electrode polarization. Against this background, the stimulation signals are transmitted as pulses.

In the subretinal implant according to aforementioned WO 2005/000395, the incident light is therefore converted into voltage pulses with a pulse length of ca. 500 microseconds and with a pulse interval of preferably 50 milliseconds, resulting in a repetition frequency of 20 Hz, which has proven sufficient for flicker-free vision. The pulse interval is also sufficient to completely return the electrode polarization. It will be noted here that 20 Hz corresponds to the physiological flicker frequency at a low surrounding brightness.

Humayun et al., "Pattern Electrical Stimulation of the Human Retina", Vision Research 39 (1999) 2569-2576, report on experiments with epiretinal stimulation, using biphasic pulses that have a cathodic phase, an interim phase and an anodic phase, each of 2 milliseconds. At a stimulation frequency of between 40 and 50 Hz, that is to say far above the physiological flicker frequency, it was possible to observe flicker-free perception in two patients.

Jensen et al., "Responses of Rabbit Retinal Ganglion Cells to Electrical Stimulation with an Epiretinal Electrode", J. Neural Eng. 2 (2005) 16-21, report on the epiretinal excitation of ganglion cells in a rabbit. With anodic and cathodic current pulses of 1 millisecond in length, they observe for excitation on the inner retina mean latency times of the ganglion cells of between 11 and 25 milliseconds.

Lovell et al., "Advances in Retinal Neuroprosthetics", in Neural Engineering, M. Aky ed.: Wiley Press, 2005, also report that the stimulation signals have to be delivered with a frequency that is appreciably greater than that required for flicker-free vision on an intact retina.

Jensen and Rizzo, "Thresholds for Activation of Rabbit Retinal Ganglion Cells with a Subretinal Electrode", Experimental Eye Research 2006, 367-373, report on subretinal stimulation experiments on an isolated rabbit retina with monophasic current pulses of 0.1 millisecond to 50 milliseconds in length, for which they observe latency times of approximately 25 milliseconds.

US 2004/019232 A1 discloses a so-called visual restoration aiding device having a plurality of electrodes placed on or under the retina of an eye. The device further comprises a photographing unit which photographs an object to be recognized by the patient, a converting unit which converts photographic data transmitted from the photographing unit to data for electrical stimulation pulse signals, and a control unit which outputs an electrical stimulation pulse signal through each electrode.

The control unit controls the signal output such that the electrical stimulation pulse signals are not simultaneously outputted to the electrodes in order to avoid interference of signals between electrodes arranged at high density. It is suggested that stimulation pulse signals simultaneously outputted through adjacent electrodes will interfere with each other, whereas stimulation pulse signals simultaneously outputted through unadjacent electrodes will not interfere with each other.

In order to allow a patient to recognize a moving image without frame dropouts, the converting rate of the object to be recognized has to be 24 to 30 Hz or more.

This device is not an active retina implant, but a camera system having an internal unit just for contacting cells of the retina, and a larger external unit for converting photographed images into electrical stimulation pulse signals.

EP 1 618 922 A1, mentioned at the outside, also discloses a camera system having an internal unit for contacting cells of the retina, this unit being provided with a limited, small number of stimulation electrodes. The number of electrodes is less than the number of electrodes needed for simultaneously outputting the stimulation signals for one frame to be viewed by the patient. Therefore, the frame is divided into sections and the internal unit is configured to sequentially output pulsed signals at predetermined time intervals, which signals only reflect a section of the frame to be viewed. In each section of a frame all electrodes may be used for outputting stimulation signals.

The article by Suaning, also mentioned at the outset, also discloses a camera system having an internal unit for contacting cells of the retina, this unit being provided with a large number of stimulation electrodes. The electrodes are divided into clusters of 7 electrodes, and into meta clusters that contain many clusters that together contain only a part of all electrodes present. One electrode my be assigned to up to 7 meta clusters. The different meta clusters are multiplexed in less than 205 us, i.e. with a very high repetition frequency, since the authors are of the opinion that for flicker free perception of patients with such an retinal implant the repetition frequency for each pixel must be increased to substantially higher than 50 Hz.

Based on a protocol approved by the competent ethics committee, and with the participation of two patients who had given their informed consent, the inventors of the present application have now performed subretinal implantation of active retina implants of the type mentioned at the outset and have examined, among other things, what effect different repetition frequencies and pulse lengths have on the visual impression. For this purpose, the implant contained a raster of electrodes that were to be directly stimulated and were at a distance of 280 micrometres from one another. The pulse shape, pulse length and pulse repetition frequency were able to be adjusted individually with the aid of an external electronics system. These as yet unpublished experiments revealed the following:
When an electrode is used for subretinal stimulation of the retina of a blind patient with biphasic, initially anodic pulses of up to 4 milliseconds in duration, and when different repetition frequencies are applied, that is to say an excitation with a constant sequence of "flashes" of defined frequency, this results in the following observation regarding the sensitivity of the patients:
At high frequencies, for example above 10 Hz, the patient senses flashes for only a short length of time, after which the perception of the flashes disappears subjectively.

In the case of an electrical stimulation with a mean frequency below 10 Hz, the stimulation impulses are, by contrast, perceived for at least a few seconds as separate flashes.

By contrast, at frequencies of a few Hz and below, each flash is sensed as an individual flash, and the sensation also remains stable for minutes.

In view of the above, the object of the present invention is to improve the construction and control of the known retina implant in such a way that it takes these observations into account and allows the patient satisfactory perception.

According to the invention, this object is achieved, in the active retina implant mentioned at the outset, by the fact that the multiplicity of stimulation electrodes are divided into at least two groups of stimulation electrodes that are triggered in chronological succession to emit stimulation signals, whereby each group of stimulation electrodes is controlled in such a way that they emit stimulation impulses with a pulse duration of less than 5 milliseconds, preferably about 0.5 millisecond, and with a repetition frequency of less than 20 Hz.

Therefore, the image seen is not imaged in its entirety onto the stimulation electrodes with a high repetition frequency; rather, the image is as it were divided into at least two partial images that are alternately "put through" to the stimulation electrodes with a lower repetition frequency.

If, for example, four partial images are emitted, each with a repetition frequency of 5 Hz, as stimulation signals from in each case one quarter of the stimulation electrodes, a new (partial) image is still emitted with a partial image frequency of in each case 20 Hz, in the form of stimulation signals, that is to say pulses, from the stimulation electrodes to the cells of the retina.

The spatial resolution is possibly slightly reduced by this, but the image repetition frequency of 20 Hz required for physiologically flicker-free vision is nonetheless achieved.

Depending on the number and spatial "density" of the stimulation electrodes, it is of course possible to use a larger number of partial images than two or four, provided that the desired spatial resolution is achieved. With a higher number of partial images, the repetition frequency of the individual partial image can then be reduced still further, but a new partial image in the form of a pattern of stimulation impulses is still emitted every 50 milliseconds, that is to say with an image repetition frequency of 20 Hz.

However, the afterglow of the triggered phosphenes provides an image impression with reduced subjective flickering.

The object of the invention is achieved in full in this way.

It is preferable that four groups of stimulation electrodes are present.,

It is also preferred if the repetition frequency of each group of stimulation electrodes is between approximately 0.2 Hz and approximately 6 Hz.

Finally, it is preferred if the groups of stimulation electrodes are interlaced in a mosaic pattern, irregularly, in lines or in columns, or are offset from one another in each case by at least one stimulation electrode.

Without being bound to the following explanation, it may be supposed that, after approximately one or two stimulations at a repetition frequency of 20 Hz in the eye, a kind of continuing depolarization or hyper-polarization occurs which, in the ganglion cells of the human retina constructed as proportional differential sensors or in the neurons of the cortex, then leads to an abatement of the sensation. This could be the same as in the healthy retina, in which a point of light projected continuously onto one and the same site of the retina also disappears subjectively.

As has already been mentioned, in the case of an electrical stimulation with a mean frequency of between 1 Hz and 5 Hz of the stimulation impulses, separate flashes are perceived at least for a few seconds. According to the findings of the inventors of the present application, this can be made use of to elicit a lasting sensation during normal eye movement, since the eye movement ensures that different areas of the retina are always being excited again.

Even if the neuronal image restoration, caused by electrical stimulation, lies in the region of 1 Hz, this low frequency can then be increased by exploiting the ability of the brain to sense the position of an object as being positionally fixed despite constant eye movement (saccades).

The current position of the eye is in fact reported to the brain which, based on this information concerning the saccades, compensates for the constantly shifting location of the object (retinal slip).

Because of the constant eye movements and the saccades, the projections of the viewed object permanently "race" around in the brain, as a result of which other neuron groups are constantly being used to identify the same object, while on the other hand, however, the subjective "fixed position" of the viewed object is achieved by the fact that the brain constantly measures the eye movements and compensates for the shifting of the object by calculation of the eye movement.

If the retina implant is implanted in the eye so as to be non-displaceable relative to the neuronal cells, such that the receiver field, that is to say the array of photodiodes, receiving the image is at all times correctly displaced along with the natural movement of the eye, that is to say with the voluntary and involuntary saccades, a consecutive frequency of several hertz can be established for the partial images, although an image is conveyed via the respective retinal neurons only in the region of 1 Hz. In the biological system, a constant restoration then takes place, occasioned by the constant eye movement by using differential cortical neuron groups.

In other words, by the permanent shifting of the visual image on the recipient field, that is to say the stimulation chip in the retinal plane, the perceived stimulation frequency can then be increased to approximately 10 Hz, which, with the inventive subdivision of the multiplicity of stimulation electrodes into at least two groups, corresponds to an image sequence of 20 Hz.

The invention is therefore based, among other things, on the realization that, in order to ensure permanent image transmission, a retina implant which is coupled anywhere to the visual pathway, and is electrically operated and stimulates nerve cells, must be implanted in such a way that the retina implant is coupled to the natural eye movement of the patient and the resulting "image shift".

The different groups of stimulation electrodes can in this case be interlaced in a mosaic pattern, the individual partial images being excited at time intervals. In this way, a technically inherent retinal slip is already built in on the retinal plane, such that new neuron groups are always being used.

In addition to a mosaic-like interlacing of the individual groups of stimulation electrodes, that is to say of the partial images, they can also be provided in lines or columns, such that the even-number lines and columns and then the odd-number lines or columns are triggered.

It is also possible to divide the array of stimulation electrodes into many spatial subunits, of which each subunit comprises, for example, four stimulation electrodes arranged in the corners of a square or rhombus, one of the four electrodes being triggered in each partial image.

Just this division of the partial images such that another of the adjacent stimulation electrodes is triggered from partial image to partial image can also improve the spatial resolution by the chronologically staggered excitation of adjacent points.

If two immediately adjacent stimulation electrodes "fire" at the same time, this may in some cases not be spatially resolved in the brain, whereas spatial resolution is possible if the closely adjacent stimulation electrodes emit their respective impulse in chronological succession.

The mode of operation according to the invention permits, for the first time, a subretinal implant which processes images with spatial resolution and which allows vision with reduced subjective flickering, and with the natural movement of the eye being used to permit the required refreshment of the neuronal cells.

Further advantages will become clear from the description and from the attached drawing.

It will be appreciated that the abovementioned features and the features still to be explained below can be used not only in the respectively cited combination but also in other combinations or singly, without departing from the scope of the present invention.

An embodiment of the invention is explained in more detail in the following description and is depicted in the drawing, in which:
- Fig. 1: shows a schematic representation of a retina implant, in a view not true to scale;
- Fig. 2: shows a schematic representation of a human eye into which the retina implant according to Fig. 1 is inserted, once again not true to scale;
- Fig. 3: shows another retina implant, again not true to scale;
- Fig. 4: shows a schematic representation of a human eye as in Fig. 1, but with the retina implant according to Fig. 3; and
- Fig. 5: shows an enlarged schematic representation of the stimulation chip from Fig. 1 or Fig. 3.

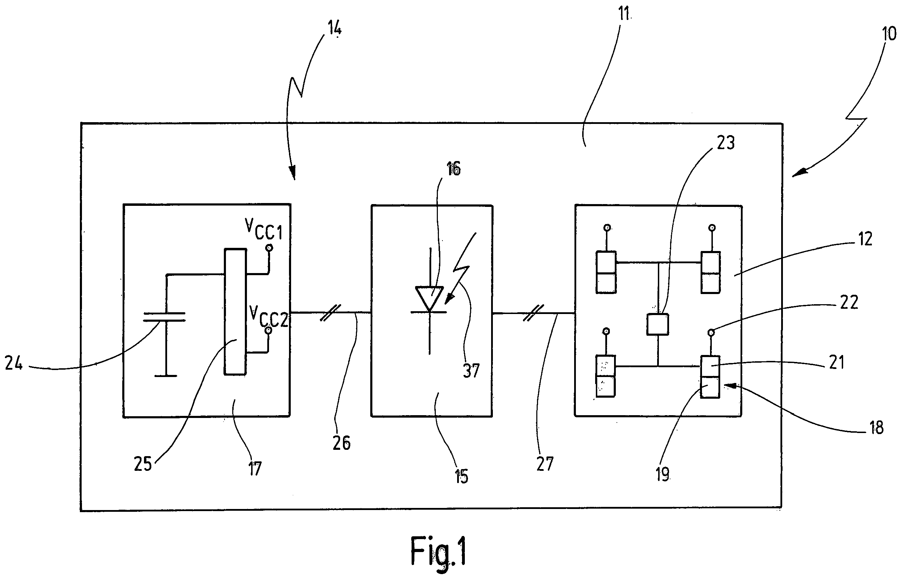

In Fig. 1, an active retina implant 10 is shown schematically, with the dimensions not true to scale.

The retina implant 10 is formed on a flexible film 11 on which a stimulation chip 12 and an energy supply 14 are arranged. The energy supply 14 comprises an IR receiver 15, which contains one or more photovoltaic elements 16 that convert incident IR light into electrical voltage. The external energy that is coupled-in in this way is transferred to a voltage supply 17.

The stimulation chip 12 comprises image elements 18 which are arranged, for example, in rows and columns and of which only four are shown in Fig. 1, for the sake of clarity. Each image element 18 comprises a logarithmic image cell 19 for local image brightness, and an amplifier 21 which is connected, at its output, to a stimulation electrode 22. The stimulation chip 12 also comprises an image cell 23 which is provided for global brightness and which is connected to the amplifiers 21 of all the image elements 18 on the stimulation chip 12. It will be understood that the stimulation chip 12 can comprise a plurality of global image cells 23, or also just one of them.

The voltage supply 17 comprises a storage element 24 in which the external energy taken up from the IR receiver 15 is stored. The storage element 24 is connected to a switch component 25 that generates two different voltage sources V_{cc1} and V_{cc2} in a manner described in greater detail below. The voltage supply 17, the IR receiver 15 and the stimulation chip 12 are connected to one another via lines 26 and 27.

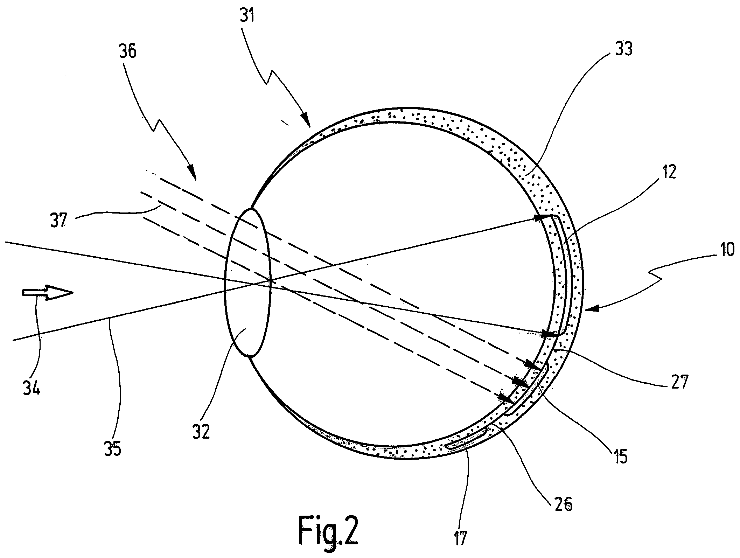

The retina implant 10 from Fig. 1 is intended to be implanted into a human eye 31, which is shown very schematically in Fig. 2. For the sake of simplicity, the latter only shows the lens 32, and the retina 33 into which the implant 10 has been implanted. The implant 10 is preferably introduced into the so-called subretinal space forming between the pigment epithelium and the photoreceptor layer. If the photoreceptor layer is degenerated or absent, the subretinal space is formed between the pigment epithelium and the layer of bipolar and horizontal cells. The retina implant 10 is positioned in such a way that stimulation signals can be applied to cells in the retina 33 via the stimulation electrodes 22 shown in Fig. 1.

Visible light, which is indicated by an arrow 34 and whose beam path can be seen at 35, is conveyed via the lens 32 onto the stimulation chip 12, where the visible light 34 is converted into electrical signals, which are converted into stimulation signals via the amplifiers 21 from Fig. 1.

It will be seen from Fig. 2 that the IR receiver 15 lies outside the area of incidence of the visible light 34. External energy 36 is directed towards the IR receiver 15 in the form of rays of IR light 37, which is converted in the IR receiver into an electrical voltage that passes first through the lines 26 to the voltage supply 17, from which corresponding supply voltages are generated. These supply voltages then pass through the lines 26 and 27 to the stimulation chip 12, where they are used to convert the incident visible light 34 into stimulation signals, in a manner described in more detail below.

The spatial separation of stimulation chip 12 and IR receiver 15 provides spatial uncoupling, such that the undesired impairment of the image cells in the stimulation chip 12 by the IR light 37 is kept low.

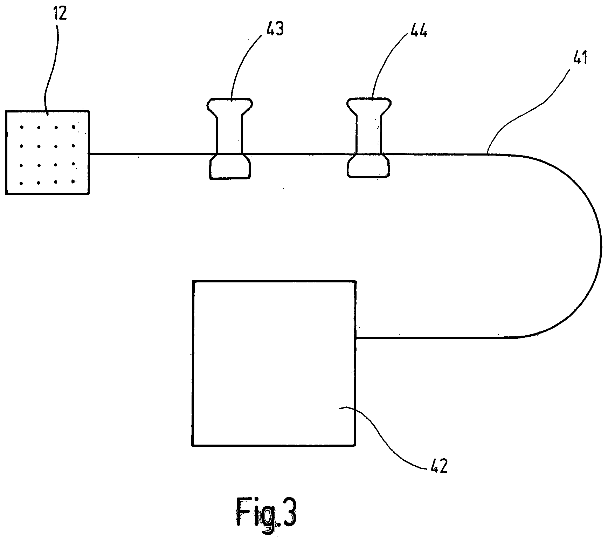

Fig. 3 shows another retina implant, in a depiction not true to scale, in which the energy supply is not effected via incident IR light, but via a connection cable 41 that connects the stimulation chip 12 to an external attachment part 42, which is fastened outside the eye, for example on the patient's skull. Electrical energy is sent to the stimulation chip 12 via the attachment part 42, while, at the same time, control signals can also be transmitted that influence the mode of operation of the stimulation chip in the manner described, for example, in the aforementioned document WO 2005/000395 A1, the content of which is herewith incorporated into the subject matter of the present application.

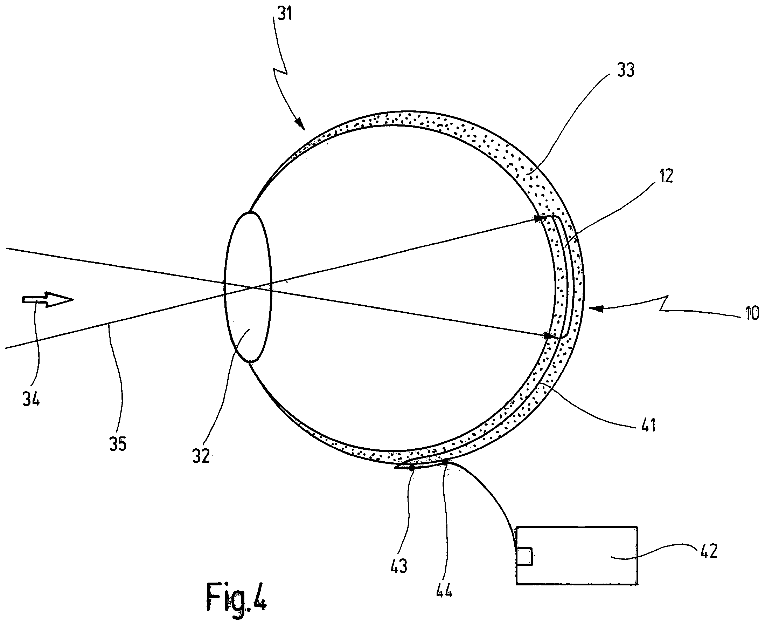

Approximately 5 cm away from the stimulation chip 12, the connection cable 41 has fastening tabs 43 and 44 with which said connection cable is secured in immovable manner on the outside on the sclera of the eye, as is shown schematically in Fig. 4.

Fig. 4 shows the same view as in Fig. 2, but with the retina implant according to Fig. 3 now implanted. It will be seen that the cable 41 is routed laterally out of the eye and is secured there on the outside on the sclera via the fastening tabs 43 and 44 before the cable continues to the external attachment part 42.

It is thereby ensured that, during movements of the eye 31, the stimulation chip 12 is held captively in the retina 33, such that it follows the saccades without shifting relative to the nerve cells of the retina 33.

It will also be noted that the dimensions in Figures 3 and 4, in particular of the stimulation chip 12, of the fastening tabs 43, 44 and of the external attachment part 42, are not shown to scale, nor are they in the correct proportion to one another.

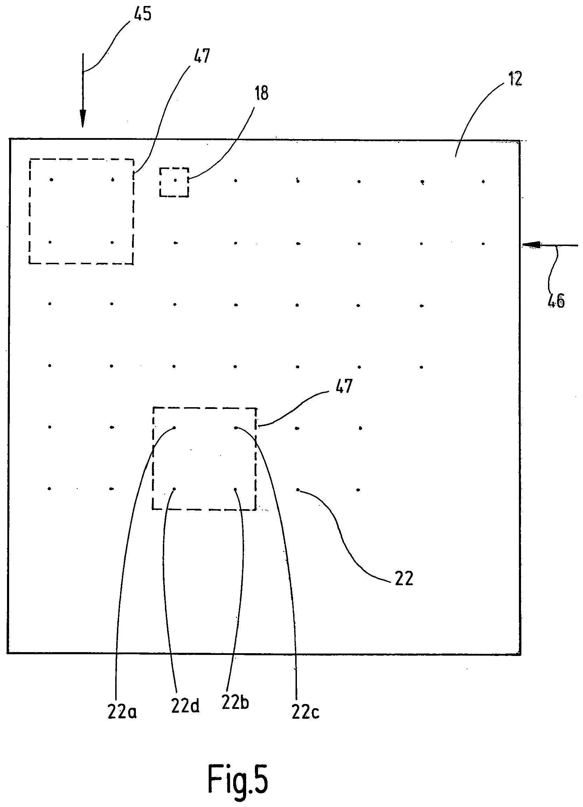

In Fig. 5, the stimulation chip 12, as used both for the retina implant from Fig. 1 and also for the retina implant from Fig. 3, is shown schematically and on an enlarged scale. As has already been mentioned at the outset, the stimulation chip 12 comprises image elements 18 that are arranged in rows 45 and columns 46 and that each contain, among other things, a stimulation electrode 22. In Fig. 5, some of the stimulation electrodes 22 are shown purely by way of example and have now been combined, according to the invention, into subgroups that are excited in chronological succession or interlaced one within another, with the result that, at any one time, only some of the electrodes 22 are connected through to emit a voltage pulse, it further being assumed, of course, that this stimulation electrode 22 is also assigned an active, that is to say illuminated image element 18. Within a partial image emitted by a subgroup of stimulation electrodes 22, the only stimulation electrodes 22 that are connected in order to emit a voltage pulse are of course the ones with corresponding image information in the received image.

The stimulation electrodes 22 in the individual subgroups or partial images can now be distributed, either randomly or regularly, in mosaic fashion relative to the total number of stimulation electrodes, and it is also possible to divide the partial images onto different columns 45 and/or rows 46.

If, for example, two partial images are used, one partial image comprises all the even columns 45 or lines 46, while the other partial image comprises all the odd columns 45 or lines 46.

It is also possible, for example, to provide four partial images, which differ from one another in each case by one stimulation electrode distance.

If, for example, four subgroups are provided, these can form different groups 47 of pixels in which groups the stimulation electrodes 22a, 22b, 22c and 22d in the four corners of the group 47 are each assigned to different subgroups. Thus, in the first partial image, the stimulation electrodes 22a at the top left in each group 47 would be triggered, if corresponding image information reaches them, and then, in the second partial image, the stimulation electrodes 22b arranged at the top right in the group, and so one.

As already mentioned at the outset, the stimulation electrodes 22 have a center spacing of each 280 µm. Each electrode 22 may be comprised of one single electrode having a respective area, or of an array of several electrodes, preferably an array of 2 x 2 electrodes, each electrode having an area of 50 x 50 µm. These four "sub-electrodes" perform the function of one stimulation electrode 22, but the smaller sub-electrodes having the advantage that these are mechanically more stable, as they do not chip-off so easily from the film 11 than a larger electrode.

## Claims

1. Active retina implant (10) for implantation into an eye, preferably into the subretinal space of an eye, with a multiplicity of stimulation electrodes (22) that emit electrical stimulation signals to cells of the retina that are to be contacted, and with a multiplicity of image elements (18) that convert incident light into the stimulation signals, **characterised by** a multiplicity of stimulation electrodes divided into at least two groups of stimulation electrodes that are triggered in such a way as to emit stimulation signals in chronological succession, and wherein each group of stimulation elements is triggered, when light impinges on the associated image elements, in such a way that they emit stimulation impulses with a pulse duration of less than 5 milliseconds, and with a repetition frequency of less than 20 Hz.

2. Retina implant according to Claim 1, **characterized in that** four groups of stimulation electrodes are present.

3. Retina implant according to Claim 1 or 2, **characterized in that** each group of stimulation elements is triggered, when light impinges on the associated image elements, in such a way that they emit stimulation impulses with a pulse duration of about 0.5 millisecond.

4. Retina implant according to Claim 3, **characterized in that** the repetition frequency of each group of stimulation electrodes is between approximately 0.2 Hz and approximately 6 Hz.

5. Retina implant according to anyone of Claims 1 to 4, **characterized in that** the groups of stimulation electrodes are interlaced in a mosaic pattern, irregularly, in lines or in columns, or are offset from one another each by at least one stimulation electrode.

6. Retina implant according to anyone of Claims 1 to 6, **characterized in that** it is provided with attachment points by way of which it can be connected to the retina in a non-displaceable manner.

## Patentansprüche

1. Aktives Retina-Implantat (10) zur Implantation in ein Auge, vorzugsweise in den sub-retinalen Raum eines Auges, mit einer Vielzahl von Stimulationselektroden (22), die elektrische Stimulationssignale an zu kontaktierende Zellen der Retina abgeben, und mit einer Vielzahl von Bildelementen (18), die einfallendes Licht in die Stimulationssignale umwandeln,
**gekennzeichnet durch** Vielzahl von in zumindest zwei Gruppen von Stimulationselektroden aufgeteilte Stimulationselektroden, die so angesteuert werden, dass sie zeitlich nacheinander Stimulationssignale abgeben, und wobei jede Gruppe von Stimulationselektroden bei Lichteinfall auf die zugeordneten Bildelemente derart angesteuert wird, dass sie Stimulationsimpulse mit einer Pulsdauer von weniger als 5 Millisekunden und einer Wiederholfrequenz geringer als 20 Hz abgeben.

2. Retina-Implantat nach Anspruch 1, **dadurch gekennzeichnet, dass** vier Gruppen von Stimulationselektroden vorhanden sind.

3. Retina-Implantat nach Anspruch 1 oder 2, **dadurch gekennzeichnet, dass** jede Gruppe von Stimulationselektroden bei Lichteinfall auf die zugeordneten Bildelemente derart angesteuert wird, dass sie Stimulationsimpulse mit einer Pulsdauer von etwa 0,5 Millisekunden abgeben.

4. Retina-Implantat nach Anspruch 3, **dadurch gekennzeichnet, dass** die Wiederholfrequenz einer jeden Gruppe von Stimulationselektroden zwischen etwa 0,2 Hz und etwa 6 Hz liegt.

5. Retina-Implantat nach einem der Ansprüche 1 bis 4, **dadurch gekennzeichnet, dass** die Gruppen von Stimulationselektroden mosaikartig, unregelmäßig, zeilenweise oder spaltenweise verschachtelt, oder zueinander jeweils um mindestens eine Stimulationselektrode versetzt angeordnet sind.

6. Retina-Implantat nach einem der Ansprüche 1 bis 5, **dadurch gekennzeichnet, dass** es mit Befestigungspunkten versehen ist, über die es unverschiebbar mit der Retina verbindbar ist.

## Revendications

1. Implant rétinien actif (10) à implanter dans un oeil, de préférence dans l'espace sous-rétinien d'un oeil, ayant une multiplicité d'électrodes de stimulation (22) qui émettent des signaux de stimulation électrique vers les cellules de la rétine avec lesquelles un contact doit être établi, et ayant une multiplicité d'éléments d'image (18) qui convertissent la lumière incidente en signaux de stimulation,
**caractérisé par** une multiplicité d'électrodes de stimulation divisées en au moins deux groupes d'électrodes de stimulation qui sont déclenchées de manière à émettre des signaux de stimulation dans l'ordre chronologique, et où chaque groupe d'éléments de stimulation est déclenché, lorsque la lumière arrive sur les éléments d'image associés, de sorte qu'ils émettent des impulsions de stimulation avec une durée d'impulsion inférieure à 5 millisecondes, et avec une fréquence de répétition inférieure à 20 Hz.

2. Implant rétinien selon la revendication 1, **caractérisé en ce que** quatre groupes d'électrodes de stimulation sont présents.

3. Implant rétinien selon la revendication 1 ou 2, **caractérisé en ce que** chaque groupe d'éléments de stimulation est déclenché lorsque la lumière arrive sur les éléments d'image associés, de sorte qu'ils émettent des impulsions de stimulation avec une durée d'impulsion d'environ 0,5 milliseconde.

4. Implant rétinien selon la revendication 3, **caractérisé en ce que** la fréquence de répétition de chaque groupe d'électrodes de stimulation est comprise entre environ 0,2 Hz et environ 6 Hz.

5. Implant rétinien selon l'une quelconque des revendications 1 à 4, **caractérisé en ce que** les groupes d'électrodes de stimulation sont entrelacés selon un motif en mosaïque, de manière irrégulière, en lignes ou en colonnes, ou sont décalés les uns par rapport aux autres par au moins une électrode de stimulation.

6. Implant rétinien selon l'une quelconque des revendications 1 à 5, **caractérisé en ce qu'**il est pourvu de points de fixation au moyen desquels il peut être relié à la rétine de manière à rester immobile.
